# EUROPEAN PATENT APPLICATION

(11) **EP 3 671 213 A1**
(43) Date of publication of application: **24.06.2020**
(21) Application number: 18214811.4
(22) Date of filing: 20.12.2018
(51) Int. Cl.: G01N 33/574, C12Q 1/6886

(54) **IN VITRO METHOD FOR THE DIAGNOSIS OF MALIGNANT CONDITIONS**

(71) Applicant: Kromat Muszerforgalmazó Kft., 1112 Budapest (HU)
(72) Inventor: Márk, László, 7623 Pécs (HU)
(74) Representative: Danubia Patent & Law Office LLC

(57) **Abstract**

An *in vitro,* non-invasive method is provided for the diagnosis of cancer, in particular of oral cancer. The method comprises quantifying a plurality of components with a molecular weight lower than a predetermined threshold in a saliva sample obtained from a subject and comparing the so obtained quantification value to a reference.

## Description

### FIELD OF THE INVENTION

The invention relates to the field of *in vitro* diagnostics. Particularly, an *in vitro,* non-invasive method is provided for the diagnosis of cancer, in particular of oral cancer. The method comprises quantifying a plurality of components with a molecular weight lower than a predetermined threshold in a saliva sample obtained from a subject and comparing the so obtained quantification value to a reference.

### BACKGROUND OF THE INVENTION

Identification of potential biomarkers to detect cancer has delivered a number of candidates which may be used in early diagnosis of the disease. More recently identification of biomarker patterns, especially polypeptide patterns characteristic of cancer or a certain type of cancer has been the focus of interest. US20070087392 describes a method for diagnosing head and neck squamous cell carcinoma, wherein a variety of protein biomarkers is detected. The protein biomarkers are differentially expressed in patients suffering from the disease. The molecular weight of the biomarkers ranges from about 2700 Da to about 15 000 Da.

Shikha Saxena et al. (A Review of Salivary Biomarker: A Tool for Early Oral Cancer Diagnosis. Adv Biomed Res. 2017; 6: 90.) have reviewed salivary biomarkers in different tumors, such as breast cancer, hepatocellular carcinoma, pancreatic cancer and oral cancer, showing that differential expression of one or more than one given polypeptide may be characteristic of the given tumour.

Chaiyarit et al. Comparative evaluation of 5-15-kDa salivary proteins from patients with different oral diseases by MALDI-TOF/TOF mass spectrometry (Clin Oral Investig. 2015 Apr;19(3):729-37) have identified two mass signals (5 592,26 Da and 8301.46 Da) using MALDI-TOF/TOF mass spectrometry, which were significantly higher in oral cancer patients than in control subjects and a specific mass signal of 7.6 kDa that was increased in oral cancer patients. The authors suggest that the peptide identity of the mass signals should be determined to validate them as biomarkers suitable for detecting oral diseases.

Saliva has been shown to contain multiple kinds of molecular and microbial analytes and biomarkers (Yoshizawa JM et al Salivary biomarkers: toward future clinical and diagnostic utilities. Clin Microbiol Rev. 2013 Oct;26(4):781-91.) Furthermore, whole saliva sample is easy to collect and is a promising tool in non-invasive diagnostic methods.

### BRIEF DESCRIPTION OF THE INVENTION

An *in vitro,* non-invasive method is provided for diagnosing cancer. In certain embodiments it is not necessary to identify the chemical structure (e.g. amino acid sequence) of a component or components, making the method simple and enabling the assessment of a huge number of samples within a short time.

*In vitro* method for the detection of tissue alterations associated with cancer in a subject, the method comprising
A) quantifying, in a saliva sample obtained from the subject,
   i) a plurality of components having a lower molecular weight than a predetermined threshold, thereby obtaining an absolute quantification value and/or
   ii) a plurality of components having a lower molecular weight than a predetermined threshold relative to a component or a plurality of components having a higher molecular weight than the predetermined threshold, thereby obtaining a relative quantification value,
B) identifying the subject as having a tissue alteration associated with cancer if the absolute and/or relative quantification value determined in step A) is higher than an absolute reference quantification value or a relative reference quantification value, respectively, wherein said predetermined threshold value is obtained by determining a characteristic molecular weight above which the amount of a plurality of components in the saliva of subjects having tissue alterations associated with cancer is lower than in the saliva of subjects free of such tissue alterations and determining said threshold value to be the same or lower than said characteristic molecular weight, and
wherein said absolute and/or relative reference quantification values are obtained by executing step Ai) and/or Aii), respectively on saliva samples obtained from an appropriate number of patients free from said tissue alterations and patients having such tissue alterations and determining the absolute and/or relative maximum quantification reference value characteristic for said patients free from said tissue alterations.

The absolute and/or relative maximum reference quantification values determined in the reference sample may be the same as the absolute and/or relative reference quantification values.

*In vitro* method for the detection of tissue alterations associated with cancer in a subject, the method comprising
A) quantifying, in a saliva sample obtained from the subject,
   i) a plurality of components having a lower molecular weight than a predetermined threshold, thereby obtaining an absolute quantification value and/or
   ii) a plurality of components having a lower molecular weight than a predetermined threshold relative to a plurality of components having a higher molecular weight than the predetermined threshold, thereby obtaining a relative quantification value,
B) identifying the subject as having a tissue alteration associated with cancer if
   i) the absolute quantification value determined in step Ai) is higher than an absolute reference quantification value and/or
   ii) the relative quantification value determined in step Aii) is higher than a relative reference quantification value,
wherein said predetermined threshold value is obtained by determining a characteristic molecular weight above which the amount of a plurality of components in the saliva of subjects having tissue alterations associated with cancer is lower than the amount of a plurality of components in the saliva of subjects free of such tissue alterations and determining said threshold value to be the same or lower than said characteristic molecular weight, and
wherein said absolute reference quantification value and/or said relative reference quantification value are obtained by
executing step Ai) and/or Aii), respectively, on saliva samples obtained from an appropriate number of patients free from said tissue alterations and determining an absolute reference quantification value and/or a relative quantification reference value characteristic for said patients free from said tissue alterations.

Alternatively, the predetermined threshold value is obtained by determining a characteristic molecular weight, below which the amount of the plurality of components in the saliva of subjects having tissue alterations associated with cancer is higher than in the saliva of subjects free of such tissue alterations, using reference samples from patients diagnosed with cancer and reference samples from healthy subjects (i.e. from subjects not suffering from cancer).

The absolute reference quantification value and/or the relative reference quantification value characteristic for patients free from tissue alterations associated with cancer may be an upper boundary value defined based on the distribution of the quantification values typical of healthy subjects. Preferably, the absolute reference quantification value and/or the relative reference quantification value are the maximum absolute value quantification value and/or the maximum relative quantification value, respectively, measured in healthy individuals (i.e. in the samples used for obtaining the absolute reference quantification value and/or the relative reference quantification value. In a preferred embodiment the absolute reference quantification value and/or the relative reference quantification value characteristic for patients free from tissue alterations associated with cancer are values which are lower than the maximum absolute value quantification value and/or the maximum relative quantification value, respectively, measured in healthy individuals. The absolute reference quantification value and/or the relative reference quantification value characteristic for patients free from tissue alterations associated with cancer may be defined based on the distribution of the quantification values typical of healthy subjects and the distribution of the quantification values typical of cancer patients.

When obtaining the predetermined threshold, the amount of a plurality of components in the saliva of subjects having tissue alterations associated with cancer is compared the amount of a plurality of components in the saliva of subjects free of such tissue alterations in the methods herein provided. A plurality of components in a test sample is compared to the corresponding components in the reference sample (i.e. in samples from healthy subjects) or a plurality of components in a certain molecular weight range or ranges is compared to the corresponding molecular weight range or ranges in the reference sample.

In preferred embodiments said threshold is determined by obtaining and analyzing mass spectra of the components present in the saliva sample.

In preferred embodiments of the methods herein provided, the total amount or the total number of the mass signals (i.e. peaks) of the components having a lower molecular weight than a predetermined threshold are determined.

In preferred embodiments of the methods herein provided, said predetermined threshold is from about 8000 Da to about 12000 Da, preferably about 12 000 Da, more preferably about 10 000 Da or about 8000 Da or about 9000 Da.

In preferred embodiments of the methods herein provided, the component(s) are component(s) which may be quantified by using UV/VIS spectrometry at a wavelength from 180 to 800 nm, preferably from 200 nm to 300 nm, more preferably from 260 nm to 300 nm, highly preferably at about 280 nm.

In preferred embodiments the quantification is carried out at more than one wavelength or more than one range of a wavelength, and the quantification value is derived from the results of the measurements at the more than one wavelengths or the more than one ranges of a wavelength.

In preferred embodiments of the methods herein provided, the plurality of components having a lower molecular weight than a predetermined threshold are separated using a membrane filter having an appropriate molecular weight cut-off or by a membrane filter having an appropriate pore size.

In preferred embodiments of the methods herein provided, the plurality of components having a lower molecular weight than a predetermined threshold are quantified using UV spectrometry. In other preferred embodiments UV/VIS spectrometry is used. UV/VIS spectrometry provides a broader range of wavelengths that can be used for quantification of components in the sample.

An *in vitro* method for the detection of tissue alterations associated with cancer, the method comprising
I) providing a mass spectrum of a saliva sample obtained from the subject,
II) comparing the region of the provided mass spectrum showing peak(s) of detected component(s) above a predetermined threshold value to the corresponding region of a reference mass spectrum being characteristic of subjects free from such tissue alterations, and
III) identifying the subject as having tissue alterations associated with cancer if said region comprises significantly less peaks than the corresponding region in the reference spectrum,
wherein said predetermined threshold value is obtained by determining a characteristic molecular weight above which the mass spectrum of the saliva of subjects having tissue alterations associated with cancer comprises significantly less peaks than the corresponding region of the mass spectrum of saliva of subjects free of tissue alterations associated with cancer and determining said threshold value to be the same or lower than said characteristic molecular weight.

The detected components may be at least one component.

In highly preferred embodiments of the methods herein provided, the plurality of components or the detected component(s) are peptides and/or proteins. In other preferred embodiments the components are selected from peptides, polypeptides, proteins, lipids and nucleic acids.

In certain embodiments of the methods herein provided, components of the saliva sample having a molecular weight higher than about 20 000 Da, preferably higher than above 18 000 Dalton are removed before carrying out step A) or I) or are not quantified or are not assessed.

In preferred embodiments of the methods herein provided, the cancer is a head and neck cancer, preferably selected from parotid tumour, squamous cell carcinoma (SCC), laryngeal SCC, pharyngeal SCC, gingival SCC, supraglottic SCC, hypopharyngeal SCC, vocal chord SCC, oesophageal SCC.

In a preferred embodiment the tumour is cervical SCC.

In preferred embodiments a subject having a tissue alteration associated with cancer is a subject having cancer. In these embodiments the methods herein provided are for diagnosing cancer.

In preferred embodiments the subject is a mammal, highly preferably human.

In preferred embodiments when an amount of a plurality of components higher or lower in a sample of a patient having a tissue alteration associated with cancer or of a patient with cancer than the amount of the plurality of components in a reference sample, the amount is significantly higher or lower, respectively.

### BRIEF DESCRIPTION OF THE DRAWINGS

Figure 1: Representative MALDI TOF MS mass spectra of human whole saliva samples of the healthy control group
Figure 2: Representative MALDI TOF MS mass spectra of human whole saliva samples of the cancerous group

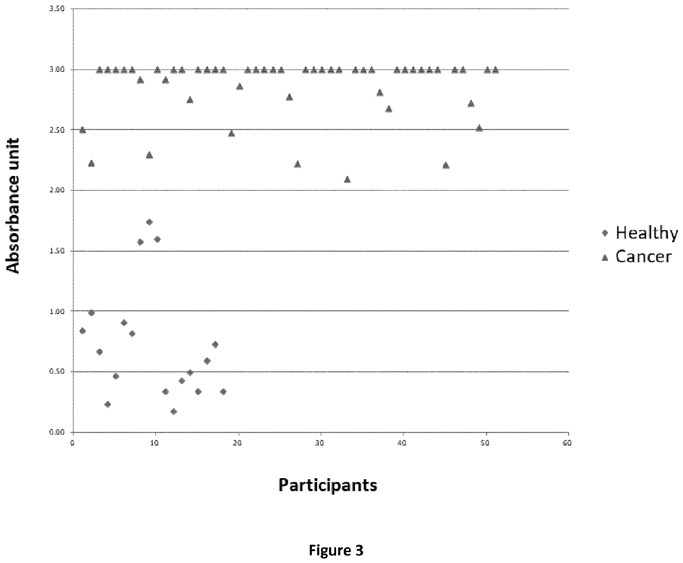

Figure 3: UV absorbance at 280 nm of saliva samples from cancer patients and healthy subjects.

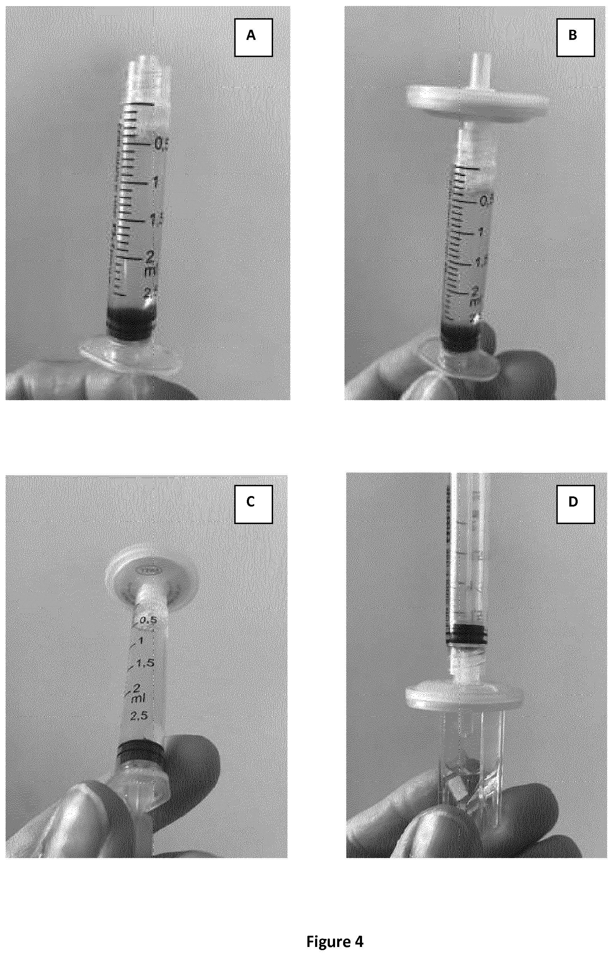

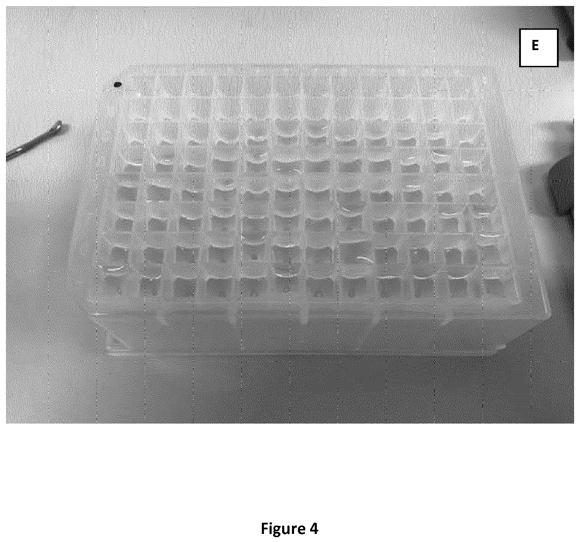

Figure 4: A sterile syringe used for sampling, B and C filter membrane on the syringe, D filtering the saliva sample directly into the microcuvette, E well plate vacuum membrane filter suitable for assessing a huge number of saliva samples simultaneously.

### DETAILED DESCRIPTION OF THE INVENTION

An *in vitro* method for the detection of tissue alterations associated with cancer in a subject is provided. Without wishing to be bound by theory, it is supposed that the method is suitable to detect tissue alterations associated with cancer by detecting degradation products of increased enzymatic activity and metabolic products obtained by an altered metabolic pathway involved in cancer.

Malignant tumours are characterised by cancer cells invading the surrounding tissues. Invasion requires the destruction of the extracellular matrix proteins by various matrix proteolytic enzymes. ECM proteins are degraded by cell-associated or extracellular enzymes secreted by both tumour cells and stromal cells.

Matrix metalloproteases (MMPs; e.g. gelatinase A, 72kDa type-IV collagenase) degrade all of the major components of the extracellular matrix (such as collagen IV, laminin, fibronectin, elastin, heparan-sulfate proteoglycans). Beside MMPs, serine proteases (e.g. urokinase-type plasminogen activator), heparanases (e.g. heparan sulfate proteoglycan (HSPG)), sulfatases and cystein peptidases are also implicated in the degradation of the ECM.

Various types of enzymes have been found to be overexpressed or mutated in different types of cancer.

Proliferating altered cells (e.g. cancer cells) produce high levels of specific proteases, thereby modifying both the serum proteome and the metabolic products thereof, i.e. the serum peptidome. Components in saliva may also be derived from degradation of the extracellular matrix, altered or increased metabolism characteristic of cancer cells. Many of the peptides in the saliva are believed to be fragments of larger proteins that have been at least partially degraded by various enzymes such as metalloproteases.

Various types of enzymes have been found to be overexpressed or mutated in different types of cancer. For example, AP-N/CD13 is highly expressed in bladder, gastric, thyroid, and hepatic carcinomas and aminopeptidase P (AP-P), and enkephalin-degrading tyrosyl aminopeptidase (EDA) have been associated with breast cancer, PSMA is overexpressed in prostate cancer.

Altered metabolic activity and biosynthesis of lipids and other macromolecules of cancer cells, which are necessary for the high rate of proliferation, are also well known. The synthesis or degradation of lipids, carbohydrates, hormones, nucleosides, nucleotides, RNA, DNA, etc. are known to be altered in cancer. Activation of oncogenic pathways stimulates e.g. lipid synthesis. Metabolic pathways that are altered in cancer are e.g. citric acid cycle, respiratory chain, glycolysis, gluconeogenesis, production and β-oxidation of fatty acids, protein degradation pathways.

Elevated lipid levels (TC, TG, HDL and LDL) in the plasma were reported in breast and other cancer patients, while other studies demonstrate a positive correlation between serum and salivary TC, TGL, HDLC, VLDLC and LDLC levels.

The terms "quantifying", "quantify" and the like refer to measuring a characteristic of a component that may be correlated with the amount of a component. Measuring such a characteristic may be carried out by measuring an indicator of the characteristic of the component that may be correlated with the amount of the component. For example a component may be quantified by measuring the concentration of the component in a sample, wherein the concentration is measured by assessing the light absorbance of the component at a given wavelength (i.e. the absorbance is used as an indicator of the concentration).

In the method provided herein the amount of a component or components is determined. The term "amount of a component" (in a sample) may relate to the concentration of the component in a sample or the total mass of the component (in the sample).

The amount of a component comprised in a sample may be determined by various methods depending on the physico-chemical (e.g. peptide, lipid, polar, apolar, amino acid sequence) or biological (e.g. enzyme activity, antigenic) characteristics of the component, the method used to separate the component from other components (e.g. separation by ultrafiltration or microfiltration membranes or identification by MS techniques). The term "determining the amount of a component" as used herein refers to determining and quantifying at least one characteristic of the component, which correlates with the amount of the component and thus may be used as an indicator of the amount present in the sample. Such characteristics may be UV absorbance of the compound on a certain wavelength, optical density, chemical reaction with an appropriate binding moiety (e.g. immunochemical binding) etc.

In certain embodiments qualitative determination of the component may also be necessary. Techniques for qualitative determination of a component are well known and vary depending on the physico-chemical and biological characteristics of the component. Examples of such techniques include immunochemistry (e.g. antigen-antibody binding), specific chemical reactions (e.g. staining), mass spectrometry. These techniques may typically be also used for quantification of the component.

In certain embodiments the absolute amount of a component or components is determined.

In certain embodiments the amount of a component or components relative to the amount of another component or components is determined.

According to the method provided herein, the amount of a plurality of components of a test sample from a subject is determined. The components have a molecular weight that is below or above of a predetermined threshold or cut-off value. It is within the skills of the skilled person to separate a component or components with a molecular weight below the predetermined threshold or cut-off value, e.g. by using appropriate molecular sieves or membranes suitable for filtration. In certain embodiments both the amount of a plurality of components with a molecular weight below the predetermined threshold or cut-off value and the amount of a component or components with a molecular weight above the predetermined threshold or cut-off value are determined. In certain embodiments a molecular sieve having an appropriate pore size is used to separate the components.

It is well within the knowledge and skills of the skilled person to select a filter with the appropriate molecular cut-off value or pore size. For example, when the threshold is from about 8 000 Da to about 12 000 Da or from about 8 000 Da to about 10 000 Da, filters with a pore size from about 0.45 µM to about 0.1 µM may be used.

The amount of the component(s) determined in the method is compared to a reference amount, i.e. the characteristic of the component that correlates with the amount of the component(s) and serves as an indicator of the amount of the component in the sample is compared to the corresponding indicator of a reference sample. The reference sample is preferably from a healthy subject(s), i.e. an individual who is not affected by the tissue alteration associated with cancer. In case the reference is from a healthy subject(s), the amount of the component(s) with the lower molecular weight than the predetermined threshold or cut-off value is indicative of cancer when said amount is higher than the reference value. More than one reference values or ranges may be determined. In that case the reference values or reference ranges are correlated with healthy or cancerous conditions. The reference value can be the average or mean or the maximum value obtained from a group of healthy subjects and/or a group of subjects suffering from cancer. The reference sample and the test sample (i.e. the sample derived from the subject that is used to detect a tissue alteration associated with cancer in the subject) are derived from saliva.

In certain embodiments the test sample is so prepared that components with a molecular weight above a predetermined value ("predetermined upper test molecular weight" as referred herein) are removed before carrying out the method herein provided. For example the test sample may be so prepared that components thereof having a molecular weight higher than about 20 kDa, preferably 18 kDa or components having a molecular weight higher than about 15 kDa are removed before carrying out the method. In such cases, before carrying out the method, the test sample does not comprise components, e.g. peptides and proteins, having a molecular weight higher than about 18 000 Da or 15 000 kDa, respectively. In other embodiments when the method of detection and determination is suitable, only components, e.g. peptides and proteins, having a molecular weight lower than the "predetermined upper test molecular weight" are examined and if desired, quantified. In preferred embodiments components with a molecular weight of below 5000 Da, preferably below 500 Da, preferably below 250 Da are not quantified or not assessed. Accordingly, only mass signals above 5000 Da, preferably above 500 Da, preferably above 250 Da may be assessed when a mass spectrum is provided. Only mass signals below 22 kDa, preferably below 20 kDa or below 18 kDa may be assessed when a mass spectrum is provided.

The term "about" as used herein and when it refers to a molecular weight value refers to a molecular weight that is in the range of ±20%, preferably in the range of ±15% of the specific value given. Thus, the term "a component with a molecular weight about 10 000 kDa" may refer to components having a molecular weight from 8 000 kDa to 12 000 kDa or from 8 500 kDa to 11 500 kDa.

The terms "tumour" and "cancer" are used interchangeably herein and refer to diseases characterised by abnormal cell growth and proliferation. Cancerous cells may invade tissues other than the tissue of their origin, i.e. cancerous cells may form a metastasis. Although the method provided herein is not limited to the detection of certain cancer or certain tissue alterations associated with certain cancer types, e.g. head and neck cancers may be detected. Head and neck cancers include oral cancers. The term "oral cancer" includes diseases characterised by any cancerous tissue growth located in the oral cavity, e.g. squamous cell carcinoma (SCC), metastatic carcinomas. The tumour is prefereably selected from parotid tumors, laryngeal SCC, pharyngeal SCC, gingival SCC, supraglottic SCC, hypopharyngeal SCC, vocal chord SCC, cervical SCC, oesophageal SCC.

The term "a plurality of' refers to more than one component. In some embodiments the total amount of the components assessed is determined, e.g. the total amount of peptides with a molecular weight below the first predetermined threshold or the total amount of components below the predetermined threshold which may be quantified at the wavelength used in the quantification steps.

### Threshold determination

A threshold value (or cut-off value) is determined before carrying out the method provided herein. A first predetermined threshold may be obtained by determining a characteristic molecular weight above which the amount of components in the saliva of subjects having tissue alterations associated with cancer is significantly lower than in the saliva of subjects free of such tissue alterations and determining said threshold value to be the same or lower than said characteristic molecular weight.

For example, mass spectra of saliva samples from tumour patients and healthy subjects - that is, subjects not suffering from cancer - are provided, e.g. by using the method described in Example 1. However, any method for providing a mass spectrum of the components in a saliva sample may be used. Based on the spectra from these two groups (cancer patients and healthy individuals), a characteristic molecular weight may be chosen. Components with a molecular weight above this characteristic molecular weight are missing or are present in a considerably lower amount in cancer patients than in healthy individuals. Thus, a threshold value may be the same or a lower value than the first characteristic molecular weight value.

The presence of a component may be indicated by the presence of one or more peaks (i.e. a mass signal) in the mass spectrum. It is also possible to determine the amount of a component that is characterised by a certain peak on the mass spectrum by methods well known in the art, see e.g. Hiraoka: Fundamentals of Mass Spectrometry, Springer, 2013. If the amount of one or more component is determined, it is possible to compare the amount of the component(s) present in the region of the provided mass spectrum above a predetermined threshold value to the amount of component(s) present in the corresponding region of the reference spectrum. The subject is identified as having tissue alterations associated with cancer or having cancer if the amount of component(s) detected in the spectrum of the subject is less than the amount of the component(s) detected in the reference spectrum.

### Reference values

One or more reference values are predetermined before carrying out the method provided herein. To determine a reference value, an appropriate number of samples are assessed, wherein the samples are obtained from an appropriate number of individuals i) free from the tissue alterations associated with cancer (i.e. healthy individuals), from an appropriate number of cancer patients (or patients with a tissue alteration associated with cancer). The skilled person is well aware of the meaning of the term "an appropriate number" when it is used in connection with a reference sample or reference value. The term "an appropriate number" refers to a plurality of samples, the number of which is suitable to derive a statistically reliable and representative reference value from said plurality of samples. In an embodiment the method provided herein is not limited to the detection of certain cancer or certain tissue alterations associated with certain cancer types. In this particular embodiment the method herein provided may be used to detect the presence of cancer or a tissue alteration associated therewith, without specifying the type of cancer. In another embodiment detection of the presence of a certain cancer (e.g. oral cancer) or a tissue alteration associated therewith is provided. In this embodiment the cancer reference values are obtained from patients suffering from said certain cancer type (e.g. oral cancer).

The skilled person will understand that in some embodiments it is not necessary to determine the chemical or physico-chemical properties of the components in the saliva samples, apart from separating the components based on their molecular weight. Thus, in these embodiments the chemical or physico-chemical properties of (e.g.) the components having a molecular weight below the first predetermined threshold are irrelevant as long as their molecular weight is below or (above, as the case may be) a predetermined threshold. The components may therefore be peptides, proteins, glucoproteins, lipid, triglycerids, carbohydrates or nucleic acids or any combinations thereof.

In a preferred embodiment the components are quantified by measuring their UV and/or visible light absorbance in a spectrophotometer (UV or UV/VIS spectrometry). A wavelength typical for a certain chemical group (e.g. peptides and proteins) may be used to quantify the components which belong to that group. In a preferred embodiment, the components to be quantified are peptides and/or proteins and a wavelength suitable to quantify peptides and/or proteins is selected (e.g. 260-300 nm, highly preferably 280 nm). In other preferred embodiments, a variety of wavelengths are selected to quantify the components and the quantification value is derived from the absorbances measured at the different wavelengths. For example, any ranges selected from the overall wavelength range of a UV or UV/VIS spectrophotometer may be used in combination with other selected ranges, as long as components to be quantified may be detected and quantified at those ranges. In preferred embodiments at least one of the ranges selected is 200-300 nm, preferably 260-300 nm, highly preferably a wavelength or range of wavelength at which peptides and/or proteins may be detected, e.g. about 280 nm.

The term "significantly" as used herein refers to a difference which is statistically significant. The statistical test to determine whether a difference is statistically significant will depend on the characteristic measured. In other embodiments the term "significantly" refers to a difference that is being considered substantial by the skilled person. For example, when a test sample contains significantly less mass signals (peaks) or amounts of components with a molecular weight above a first predetermined threshold than the mass signals of components with a molecular weight above a first predetermined threshold in a reference sample, the number of mass signals or the amounts are significantly less, when the number or amount in the test sample is at least 40%, preferably at least 50%, more preferably at least 75%, most preferably at least 85% less than the number or amount in the reference sample.

### EXAMPLES

### Example 1. Threshold determination

### Clinical samples

Briefly, participants were asked to rinse their mouth three times thoroughly with tap water before collection of saliva samples; saliva specimen of 2 ml were taken with Braun 5 ml syringes from nonstimulated oral cavity in buccal fold and stored on ice in Protein Lobind tube (Eppendorf, Wien, Austria) using LoRetention tips (Eppendorf, Wien, Austria), followed by a 4°C centrifugation at 2500 rpm for 12 minutes. Supernatant was consequently removed and stored at minus 18° Celcius for investigation.

### Mass spectrometry

The mass spectrometer used in this work was an AutoflexII TOF/TOF (Bruker Daltonics, Bremen, Germany) operated in the linear detector for MALDI TOF with an automated mode using the FlexControl software. The ions were accelerated under delayed extraction conditions (200 ns) in positive ion mode with an acceleration voltage of 20.00 kV. The instrument uses a 337 nm pulsed nitrogen laser, model MNL-205MC (LTB Lasertechnik Berlin GmbH, Berlin, Germany). External calibration was performed in each case using Bruker Peptide Calibration Standard (#206195 Peptide Calibration Standard, Bruker Daltonics, Bremen, Germany). 1 µL of the standard solutions (prepared by dissolving saturated α-cyano-4-hydroxycinnamic acid of 1.0 ml Acetonitrile/0.1% Trifluoracetonic acid 50/50 (V/V)) were loaded onto the target plate (MTP 384 target plate ground steel TF, Bruker Daltonics, Bremen, Germany)

The extracts of the saliva in Protein Lobind tube (Eppendorf, Wien, Austria) were loaded onto the target plate using LoRetention tips (Eppendorf, Wien, Austria) by mixing 1.0 µL of each solution with the same volume of a matrix solution, prepared by dissolving saturated Sinapic acid of 1.0 ml Acetonitrile/0.1% Trifluoracetonic acid 50/50 (V/V))

Each spectrum was processed by accumulating data from 500 consecutive laser shots for Peptide calibration standard solution and 1000 shots for saliva samples. The Bruker FlexControl 2.4 software was used for controlling the instrument and the Bruker FlexAnalysis 2.4 software for spectra evaluation. Protein masses were acquired with a range of m/z 5000 to m/z 18000.

### Results

Participants were divided into a cancer (diagnosed with oral cancer, SCC) and a healthy (i.e. subjects not suffering from cancer) control group. Each group contained a total of n=50 participants.

Spectra of m/z 5000 to m/z 18000 were regarded with MALDI TOF evaluation. Spectra were divided in excerpts concerning m/z 6000-8000, m/z 9000-12000 and m/z 14000-16000 in each group.

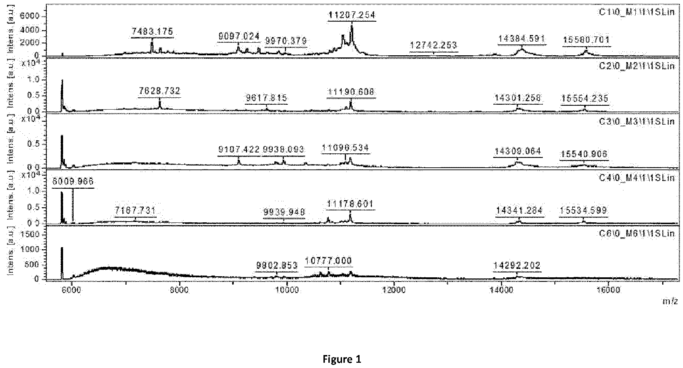

Healthy control group showed peaks in each segment. A definite presence of components, specifically at m/z 15552 (±28) and m/z 14325 (±33) in the high mass protein segment appeared (Figure 1).

The middle segment in this group showed two peaks between m/z 9617 and m/z 11207 and a further two peaked segment in between m/z 7628 and m/z 5800.

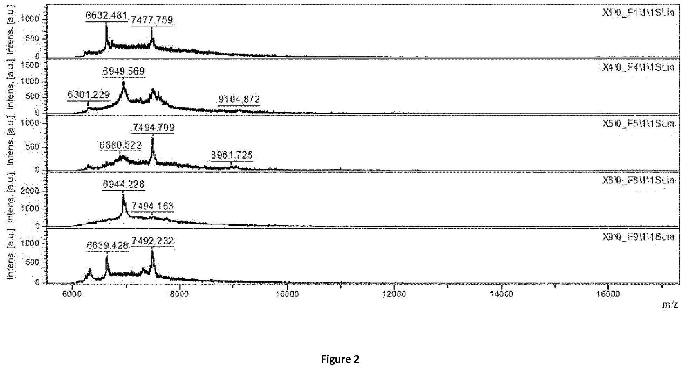

In the cancer group, changes were visible at ranges from m/z 6000 to m/z 9104 where a complete disappearance of higher mass proteins over expression was discovered. Peaks in lower protein mass segment were considerably at two mass counts differing in all regarded patients of between m/z 6639 and m/z 7494 (Figure 2).

Significant changes could be identified between control and cancerous saliva specimen. Changes in protein levels between m/z 13000 to m/z 16000 were developing to a total disappearance in cancer patients, whereas the healthy control group showed a definite appearance of those proteins. Protein levels in the range of m/z 9000 to m/z 12000 were at a minimum in cancerous individuals. The spectrum of small sized proteins in levels between m/z 6000 to m/z 8000 were significantly higher in saliva of tumorous patients. Our results show a decrease of molecular weight proteins above 9 kDa to 18 kDa.

Enzymatic cellular internal digestion or overexpression of enzymatic activity might be responsible reasons for the disappearing of (overexpressed) proteins with masses above 9 kDa and could state the higher count of lower mass proteins below 9 kDa. Higher enzymatic activity has been stated to be represented in cancer cells.

The developed method is already suitable clinically to separate cancer patients from healthy condition, as well as it is cheap and noninvasive and suitable for a high-throughput method with estimated 5000 possible samples per day.

### Example 2. UV and/or UV-VIS spectrophotometry

### Clinical samples

Briefly, participants (patients with an oral cancer diagnosis and healthy subjects (i.e. subjects not suffering from cancer)) were asked to rinse their mouth three times thoroughly with tap water be-fore collection of saliva samples; saliva specimen of 2 ml were taken with Braun 5 ml syringes from nonstimulated oral cavity in buccal fold and stored on ice in Protein Lobind tube (Eppendorf, Wien, Austria) using LoRetention tips (Eppendorf, Wien, Austria).

### Spectrophotometry

One milliliter of unstimulated saliva samples were filtered by a syringe filter membrane device with 10 kDa nominal molecular weight cut off and/or 0.1 micrometer pore size. The supernatants were collected directly into a plastic 10 mm / 1mm micro cuvette (UVette, light transmission of 200 nm - 1600 nm, Eppendorf, Wien, Austria). The spectrophotometer used in this work was an Eppendorf BioSpectrometer D30. The absorbance spectra were acquired 200-830 nm. The protein and peptide contents of the saliva samples were determined at 280 nm with factory setting of protein mode. Each sample was measured three times and three technical replicates were produced from each collected saliva.

Results are shown in table 1.

**Table 1 UV absorbance of the assessed saliva samples**

| Tumour | Healthy |
|---|---|
| 2.51 | 0.85 |
| 2.24 | 1.00 |
| 3.00 | 0.68 |
| 3.00 | 0.47 |
| 3.00 | 0.91 |
| 3.00 | 0.83 |
| 3.00 | 1.58 |
| 2.92 | 1.75 |
| 2.30 | 1.60 |
| 3.00 | 0.35 |
| 2.92 | 0.18 |
| 3.00 | 0.43 |
| 3.00 | 0.51 |
| 3.00 | 0.35 |
| 3.00 | 0.60 |
| 2.48 | 0.74 |
| 2.87 | 0.35 |
| 3.00 | |
| 3.00 | |
| 3.00 | |
| 3.00 | |
| 3.00 | |
| 2.78 | |
| 2.22 | |
| 3.00 | |
| 3.00 | |
| 3.00 | |
| 3.00 | |
| 3.00 | |
| 2.10 | |
| 3.00 | |
| 3.00 | |
| 3.00 | |
| 2.82 | |
| 2.68 | |
| 3.00 | |
| 3.00 | |
| 3.00 | |
| 3.00 | |
| 3.00 | |
| 3.00 | |
| 2.22 | |
| 3.00 | |
| 3.00 | |
| 2.73 | |
| 2.52 | |
| 3.00 | |
| 3.00 | |

## Claims

1. *In vitro* method for the detection of tissue alterations associated with cancer in a subject, the method comprising
A) quantifying, in a saliva sample obtained from the subject,
i) a plurality of components having a lower molecular weight than a predetermined threshold, thereby obtaining an absolute quantification value and/or
ii) a plurality of components having a lower molecular weight than a predetermined threshold relative to a plurality of components having a higher molecular weight than the predetermined threshold, thereby obtaining a relative quantification value,
B) identifying the subject as having a tissue alteration associated with cancer if
i) the absolute quantification value determined in step Ai) is higher than an absolute reference quantification value and/or
ii) the relative quantification value determined in step Aii) is higher than a relative reference quantification value,
wherein said predetermined threshold value is obtained by determining a characteristic molecular weight above which the amount of a plurality of components in the saliva of subjects having tissue alterations associated with cancer is lower than the amount of a plurality of components in the saliva of subjects free of such tissue alterations and determining said threshold value to be the same or lower than said characteristic molecular weight, and
wherein said absolute reference quantification value and/or said relative reference quantification value are obtained by
executing step Ai) and/or Aii), respectively, on saliva samples obtained from an appropriate number of patients free from said tissue alterations and determining an absolute reference quantification value and/or a relative quantification reference value characteristic for said patients free from said tissue alterations.

2. The method according to claim 1, wherein said predetermined threshold value is determined by obtaining and analyzing mass spectra of the components present in the saliva samples.

3. The method according to any one of the preceding claims, wherein the plurality of components are components which may be quantified by using UV/VIS spectrometry at a wavelength from 180 nm to 800 nm, preferably from 200 nm to 300 nm, more preferably from 260 nm to 300 nm, highly preferably at about 280 nm.

4. The method according to any one of the preceding claims, wherein the plurality of components having a lower molecular weight than a predetermined threshold value are separated using a membrane filter having an appropriate molecular weight cut-off or having an appropriate pore size.

5. The method according to any one of the preceding claims, wherein the plurality of components having a lower molecular weight than a predetermined threshold value are quantified using UV/VIS spectrometry, preferably UV spectrometry.

6. *In vitro* method for the detection of tissue alterations associated with cancer, the method comprising
I) providing a mass spectrum of a saliva sample obtained from the subject,
II) comparing the region of the provided mass spectrum showing peak(s) of detected component(s) above a predetermined threshold value to the corresponding region of a reference mass spectrum being characteristic of subjects free from such tissue alterations, and
III) identifying the subject as having tissue alterations associated with cancer if said region comprises significantly less peaks than the corresponding region in the reference spectrum,
wherein said predetermined threshold value is obtained by determining a characteristic molecular weight above which the mass spectrum of the saliva of subjects having tissue alterations associated with cancer comprises significantly less peaks than the corresponding region of the mass spectrum of saliva of subjects free of such tissue alterations and determining said threshold value to be the same or lower than said characteristic molecular weight.

7. The method according to any one of the preceding claims, wherein said predetermined threshold value is from about 8000 Da to about 12000 Da, preferably about 12 000 Da, more preferably about 10 000 Da or about 8000 Da.

8. The method according to any one of the preceding claims wherein the component(s) are peptide(s) and/or protein(s).

9. The method according to any one of the preceding claims, wherein components of the saliva sample having a molecular weight higher than about 20 000 Da, preferably higher than above 18 000 Dalton are removed before carrying out step A) or I) or are not assessed.

10. The method according to any one of the preceding claims, wherein the cancer is a head and neck cancer, preferably selected from parotid tumour, squamous cell carcinoma (SCC), laryngeal SCC, pharyngeal SCC, gingival SCC, supraglottic SCC, hypopharyngeal SCC, vocal chord SCC, oesophageal SCC.
